# EUROPEAN PATENT APPLICATION

(11) **EP 1 880 716 A1**
(43) Date of publication of application: **23.01.2008**
(21) Application number: 06117525.3
(22) Date of filing: 19.07.2006
(51) Int. Cl.: A61K 9/20, A61K 9/28, A61K 45/06, A61K 31/43, A61K 31/424

(54) **Kit of parts comprising an acid labile and an acid resistant pharmaceutically active ingredient**

(71) Applicant: SANDOZ AG, 4056 Basel (CH)
(72) Inventor: Raneburger, Johannes, 6300 Wörgl (AT); Schwarz, Franz, Xaver, 6300 Wörgl (AT)
(74) Representative: Dietz, Jörg-Reimar

(57) **Abstract**

The present invention relates to a combination of pharmaceutical compositions adapted for oral administration one of which comprises a pharmaceutically active ingredient being acid resistant, and the other of which comprises a pharmaceutically active ingredient being acid labile. Said combination is in the form of a kit of parts which is administered in such way as to use food intake to optimize absorption of both the acid labile and the acid resistant active ingredient. Consequently, the kit of parts of the present invention when administered according to the invention provides improved bioavailability of both the acid-resistant and acid-labile active ingredient.

## Description

### Field of the invention

The present invention relates to a combination of pharmaceutical compositions one of which comprising a pharmaceutically active ingredient being acid resistant, and another one comprising a pharmaceutically active ingredient being acid labile, which compositions are adapted for oral administration. More particularly the present invention relates to said combination in the form of a kit of parts allowing for oral administration of said compositions in such a way as to use food intake to optimize absorption of both the acid labile active ingredient and the acid resistant active ingredient and to thereby provide reliable and consistent blood levels of both active ingredients. Consequently, the kit of parts of the present invention and its use according to the invention provide improved bioavailability of both the acid-resistant and acid-labile active ingredient.

### Background of the invention

Conventional pharmaceutical compositions for oral administration comprising a combination of an acid resistant and an acid labile pharmaceutically active ingredient are usually compositions designed to comprise both active ingredients within one dosage unit, e.g. within one tablet. Alternatively, known combinations of an acid resistant and an acid labile pharmaceutically active ingredient may be in the form of more than one composition or dosage unit, e.g. in the form of different tablets, to be administered simultaneously.

If those conventional combinations are administered to patients together with or after food intake, it will be difficult to predict the influence of the ingested food on the absorption and consequently on the blood levels and hence on the bioavailability of one or of both of the active ingredients of said combination. Such influence may be disadvantageous and may further be expressed differently depending on the type of active ingredient. It has been reported for example in US patent application US 2004/0248942 A1 that food ingestion reduces bioavailability of the acid labile substance omeprazole when co-administered e.g. with sodium bicarbonate as compared to bioavailability when fasting. It may thus be difficult to control and/or optimize blood levels of an acid labile and an acid resistant active ingredient when they are administered in conventional combinations as described above after or together with food. Thus, also the therapeutic efficacy of the combined action of both active ingredients may be disadvantageously influenced by food intake. Even within one and the same patient blood levels may strongly vary from one administration to another one depending on whether the patient has had a meal or not. It is known that reliable therapeutic efficacy correlates with reliable and consistent blood and tissue levels of a given active ingredient or - in case of a combination therapy - of the active ingredients therein used.

It would therefore be desirable to develop a combination of an acid resistant and an acid labile pharmaceutically active ingredient in such a form as to minimize the influence of food intake or to even allow food intake to be used to advantageously influence absorption and/or bioavailability of both pharmaceutically active ingredients of said combination in order to provide consistent and reliable blood levels which are therapeutically effective.

### Summary of the invention

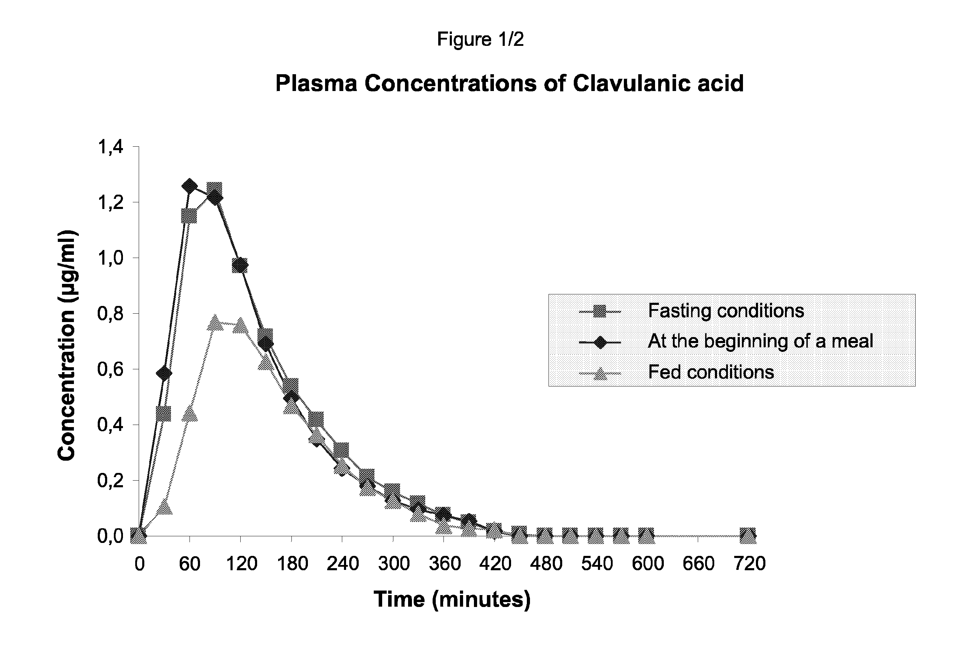

The inventors have found that an acid labile active ingredient such as clavulanic acid shows reduced absorption, and thus decreased bioavailability, when administered in combination with an acid resistant active ingredient such as amoxicillin in a fed state as is seen in Figure 1 of Comparative Example A.

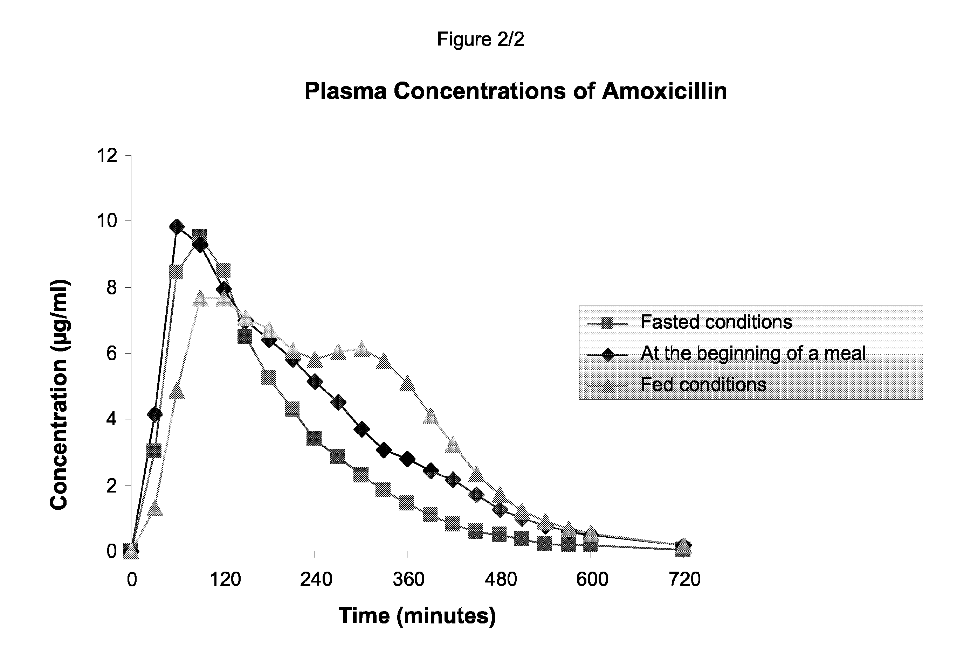

On the other hand, the inventors have observed that food intake seems to increase bioavailability of the acid resistant active ingredient within the combination, for example of amoxicillin, by causing a delayed and modified absorption showing lower maximum plasma levels, but prolonged therapeutically relevant plasma levels of amoxicillin as compared to fasting conditions as shown in Figure 2 of Comparative Example A.

The inventors have furthermore observed that food intake seems to strongly influence the in vivo disintegration behaviour of conventional compositions comprising a combination of an acid labile and an acid resistant active ingredient, for example of tablets comprising clavulanic acid and amoxicillin as described in Comparative Example B. Food intake has been found to reduce tablet erosion e.g. in the stomach.

Investigating the above mentioned tablets comprising clavulanic acid and amoxicillin, the inventors have further observed that the time point of ingestion of such conventional compositions comprising a combination of an acid labile and an acid resistant pharmaceutically active ingredient in relation to the intake of food has a significant impact on intragastric localization and disintegration and/or dissolution characteristics of said compositions which may cause the above mentioned food effects.

Based on these findings, the inventors have developed the kit of parts of the invention in an attempt to overcome the negative influences and utilize the potentially positive influences of food intake on compositions comprising a combination of an acid labile and an acid resistant active ingredient.

The present invention thus relates to a kit of parts comprising:
(A) a first pharmaceutical composition comprising an acid resistant pharmaceutically active ingredient in admixture with a pharmaceutically acceptable excipient; and
(B) a second pharmaceutical composition comprising an acid labile pharmaceutically active ingredient in admixture with a pharmaceutically acceptable excipient;
   which compositions (A) and (B) are not identical,
wherein composition (B) is to be administered before a meal and composition (A) is to be administered after a meal.

The present invention also provides the kit of parts of the invention for use as a medicament, the use of the kit of parts of the invention for the manufacture of a medicament for the prevention and/or treatment of a disease being susceptible to the combination of the acid resistant active ingredient and the acid labile active ingredient, and the use of a meal for separating the oral administration of a first composition (A) comprising an acid resistant pharmaceutically active ingredient in admixture with a pharmaceutically acceptable excipient, and of a second composition (B) comprising an acid labile pharmaceutically active ingredient in admixture with a pharmaceutically acceptable excipient wherein compositions (A) and (B) are not identical and wherein composition (B) is to be administered before composition (A). Furthermore, the present invention provides a method for preventing and/or treating a disease being susceptible to the combination of an acid resistant active ingredient and an acid labile active ingredient by administering compositions (A) and (B) of the kit of parts of the invention as herein described.

The kit of parts of the invention and its use as herein defined allow for an administration of a combination of an acid resistant and an acid labile pharmaceutically active ingredient in such a way as to use food intake to advantageously influence bioavailability of both active substances. Preferably, the kit of parts of the invention will minimize the negative influence of food intake on the absorption of the acid labile active ingredient whilst maintaining the potentially positive influence of food intake on the acid resistant active ingredient such as providing prolonged and therapeutically active blood levels of e.g. amoxicillin, particularly when incorporated into an extended release formulation.

Furthermore, the choice of distributing the total therapeutically effective amount of the acid resistant ingredient between the compositions (A) and (B) of the kit of parts and the option to use immediate and/or extended-release formulations for one or both of the compositions of the kit of parts of the invention allow to specifically "modulate" the pharmacokinetics of both active ingredients to adapt them to therapeutic needs.

### Brief description of the drawings

Figure 1: plasma levels of clavulanic acid after ingestion of an extended release tablet comprising amoxicillin and clavulanic acid, also called test tablet, in a fasting state, after food intake or at the beginning of a meal; Y-axis: plasma concentrations of clavulanic acid in µg/ml, values are mean concentrations as determined from data originating from 9 volunteers; X-axis: time in minutes after administration of the test tablet; conditions and measurement as described in Comparative Example A.
Figure 2: plasma levels of amoxicillin after ingestion of the above mentioned test tablet, in a fasting state, after food intake or at the beginning of a meal; Y-axis: concentration of amoxicillin in the blood sample in µg/ml, values are mean concentrations as determined from data originating from 9 volunteers; X-axis as described for Figure 1.

### Detailed description of the invention

The kit of parts of the invention comprises a first composition (A) comprising at least one acid resistant pharmaceutically active ingredient in admixture with a pharmaceutically acceptable excipient; and a second composition (B) comprising an acid labile pharmaceutically active ingredient in admixture with a pharmaceutically acceptable excipient; which compositions (A) and (B) are not identical, wherein composition (B) is to be administered before a meal and composition (A) is to be administered after a meal.

Composition (A) comprises at least one acid resistant pharmaceutically active ingredient such as - but not limited to - those listed below: acid resistant β-lactam antibiotics, e.g. acid resistant penicillins and derivatives thereof, such as amoxicillin, acid resistant macrolides such as clarithromycin and azithromycin; acid resistant antiepileptics such as sodium valproate; acid resistant antiphlogistics such as diclofenac sodium. Preferably said acid resistant pharmaceutical ingredient is amoxicillin or clarithromycin, or a pharmaceutically acceptable salt and/or solvate and/or hydrate thereof.

The term "acid resistant (pharmaceutically) active ingredients " as herein used comprises moderately, highly and very highly acid resistant active ingredients. Moderately acid resistant active ingredients are understood to comprise active ingredients the breakdown half-life of which is 40min or more at 37°C in aqueous solutions having a pH value of 3.0. Highly acid resistant active ingredients are understood to comprise active ingredients the breakdown half-life of which is 90min or more at 37°C in aqueous solutions having a pH value of 3.0. Very highly acid resistant active ingredients are understood to comprise active ingredients the breakdown half-life of which is 180 min or more at 37°C in aqueous solutions having a pH value of 3.0. The term "acid resistant" is understood to be synonymous to "acid stable". The term "breakdown" means the degradation of the pharmaceutically active molecule. The breakdown half-life can be measured by HPLC analysis of the breakdown kinetics. Water soluble pharmaceutically active ingredients, that is for the purpose of this invention such compounds which are soluble at a concentration of 0.2 g/I in the below-mentioned Phosphate buffer at 37°C, can be tested at said concentration of 0.2 g/I in 50mM Phosphate buffer having a pH of 3.0. For less soluble APIs a 4:1 water/acetonitril solvent system can be used, for even less soluble APIs the ration water/acetonitril can be so adapted as to solubilize the API at a concentration of 0.2g/l at 37°C at pH 3.0.

Composition (B) of the invention comprises at least one acid labile pharmaceutically active ingredient such as - but not limited to - those listed below: acid labile β-lactam antibiotics and/or other acid labile β-lactam compounds, e.g. clavulanic acid, acid labile macrolides such as erythromycin, acid labile pump inhibitors such as omeprazole, esomeprazole, lansoprazole, rabeprazole, pantoprazole, pariprazole, leminoprazole, ilaprazole, tenatoprazole; acid labile analgesics such as acetyl salicylic acid; acid labile antiphlogistic enzymatic agents such as serrapeptase and semi-alkaline proteinase, acid labile vitamins such as vitamin B₁₂, fursultiamine, folic acid, vitamin A and vitamin D. Preferably, the acid labile pharmaceutically active ingredient is clavulanic acid.

The terms "acid labile (pharmaceutically) active ingredients " as herein used are understood to comprise highly, moderately and weakly acid labile active ingredients. Weakly acid labile ingredients are understood to mean active ingredients the breakdown half-life of which is less than 30min at 37°C in aqueous solutions having a pH value of 3.0. Moderately acid labile active ingredients are understood to mean active ingredients the breakdown half-life of which is less than 15min at said conditions. Highly acid labile active ingredients are understood to mean active ingredients the breakdown half-life of which is less than 5min. The breakdown half-life can be measured by HPLC analysis of the breakdown kinetics. Water soluble pharmaceutically active ingredients, that is for the purpose of this invention such compounds which are soluble at a concentration of 0.2 g/I in the below-mentioned Phosphate buffer at 37°C, can be tested at said concentration of 0.2 g/I in 50mM Phosphate buffer having a pH of 3.0. For less soluble APls a 4:1 water/acetonitril solvent system can be used, for even less soluble APls the ration water/acetonitril can be so adapted as to solubilize the API at a concentration of 0.2g/l at 37°C at pH 3.0.

The terms _{"}pharmaceutically active ingredients" and "active ingredients" are understood to be synonymous and interchangeable.

The following pharmaceutically active ingredients may also be used in the compositions (A) and composition (B) of the kit of parts of the present invention in as far as they are to be considered as being acid labile or acid resistant according to the herein described definitions: cardiovasculars, e.g. ACE-inhibitors such as benazepril, calcium channel blockers such as amlodipine; lipid-lowering agents such as atorvastatine; antivirals, e.g. nucleoside and nucleotide reverse transcriptase inhibitors such as zidovudine, didanosine, stavudine, lamivudine, abacavir, zalcitabine, tenofovir disoproxil, adefovir dipivoxil or emtricitabine, or non-nucleoside reverse transcriptase inhibitors such as delavirdine, nevirapin or efavirenz; or protease inhibitors such as indinavir, nelfinavir, ritonavir or saquinavir, or others.

Also included are the prodrugs and the pharmaceutically acceptable salts and the solvates and hydrates of the herein described acid resistant and acid labile active ingredients. The term "prodrug" is understood to mean a compound in which the pharmacological action (active curative agent) results from conversion by metabolic processes within the body.

Preferred combinations are a weakly acid labile active ingredient in composition (B) and a moderately acid resistant, a highly acid resistant or a very highly resistant active ingredient in composition (A). Further preferred combinations are a moderately acid labile active ingredient in composition (B) and a moderately acid resistant, a highly acid resistant or a very highly resistant active ingredient in composition (A). Further preferred combinations are a highly acid labile active ingredient in composition (B) and a moderately acid resistant, a highly acid resistant or a very highly resistant active ingredient in composition (A).
Further preferred combinations are a moderately acid resistant active ingredient in composition (A) and a weakly acid labile, moderately acid labile or highly acid labile active ingredient in composition (B). Further preferred combinations are a highly acid resistant active ingredient in composition (A) and a weakly acid labile, moderately acid labile or highly acid labile active ingredient in composition (B). Further preferred combinations are a very highly acid resistant active ingredient in composition (A) and a weakly acid labile, moderately acid labile or highly acid labile active ingredient in composition (B).

Composition (A) and composition (B) may further comprise one or more pharmaceutically acceptable excipients which are preferably selected from the group consisting of fillers, sweeteners, buffering agents, glidants, flowing agents, flavouring agents, colorants, lubricants, preservatives, surfactants, wetting agents, plasticizers, binders, anticaking agents, disintegrants and thickeners, and optionally film-forming components. Other excipients known in the field of pharmaceutical compositions may also be used. Furthermore, the compositions (A) and (B) may comprise a combination of 2 or more excipients also within one of the members of the above mentioned group.

Suitable fillers are e.g. sucrose, mannitol and cellulose, e.g. microcrystalline cellulose. Suitable sweeteners are e.g. sucrose and Aspartame. Suitable buffering agents are e.g. citric acid and sodium citrate. A suitable glidant or flowing agent is e.g. colloidal silica, e.g. Aerosil^{®}. Suitable flavours are e.g. strawberry and raspberry. A suitable colorant is e.g. titan oxide. A suitable flowing agent is e.g. colloidal silicon dioxide. A suitable plasticizer is e.g. triethyl citrate. A suitable anticaking agent is e.g. talc. A suitable lubricant is e.g. magnesium stearate. A suitable preservative is e.g. sodium benzoate. A suitable surfactant and/or wetting agent is e.g. sodium lauryl sulfate. Suitable disintegrants are e.g. cross-linked polyvinylpyrrolidones, e.g. crospovidones, cross-linked sodium carboxymethylcellulose, e.g. croscarmellose sodium, calcium-carmellose or sodium starch glycolate. Suitable thickeners are e.g. xanthan gum and guar gum. Examples of suitable binders include starches and modified starches, e.g., pregelatinized starch, celluloses, e.g. hydroxypropylmethylcellulose, hydroxypropyl cellulose, hydroxyethyl cellulose and polyvinyl pyrrolidone, e.g. Povidone. Examples of film-forming components are ethylcellulose and hydroxypropyl methylcellulose.

Both composition (A) and composition (B) may exist in different dosage forms such as in the form a tablet, capsule, powder for oral suspension, granules for oral suspension, granules, pellets, dispersible tablets or in liquid form such as in the form of a suspension. In a preferred embodiment, composition (A) and composition (B) exist in the form of tablets or capsules. In another preferred embodiment, composition (A) and composition (B) exist in the same form or in different forms. Methods of preparation of such dosage forms are known such as techniques of wet or dry granulation, compaction, compression or mere mixture of components as known in the field of pharmaceutical industry.

In a further aspect, the forms in which composition (A) and composition (B) exist may themselves be differently formulated, for example one or the other may be an immediate release formulation or a controlled release and/or modified release formulation such as, but not limited to, a sustained or extended or delayed release formulation. In one preferred embodiment, composition (B) is an immediate release formulation, and composition (A) is a controlled release and/or modified release formulation, preferably an extended release formulation. Composition (A) and composition (B) may be coated, e.g. film-coated, e.g. may be immediate release coated, slow release coated, sustained release coated, delayed release coated or enteric coated, if appropriate. Techniques of preparing the herein mentioned formulations, as well as coating techniques are known.

Preferably, composition (A) and (B) are not identical in that they differ in the active substances which they comprise, and/or in the excipients, and/or in the dosage forms and/or in the formulations.

In one preferred embodiment, composition (A) comprises one acid resistant pharmaceutically active ingredient as the only active ingredient in admixture with a pharmaceutically acceptable excipient; and composition (B) comprises one acid labile pharmaceutically active ingredient as the only active ingredient in admixture with a pharmaceutically acceptable excipient.

In another preferred embodiment, composition (A) comprises an acid resistant pharmaceutically active ingredient as the only active ingredient in admixture with a pharmaceutically acceptable excipient; and composition (B) comprises an acid labile pharmaceutically active ingredient and additionally part of the acid resistant pharmaceutically active ingredient of composition (A) as the only active ingredients in admixture with a pharmaceutically acceptable excipient. In this case, composition (B) comprises the acid resistant pharmaceutically active ingredient of composition (A) in an amount which may be smaller, equal or larger than the amount present in composition (A). For example, the acid resistant pharmaceutically active ingredient may be distributed between composition (A) and (B) in a ratio of about 90% to about 10%, or of about 70% to about 30%, or of about 50% to about 50%, or of about 40% to about 60% wherein % are weight/weight percentages and relate to the total amount of the acid resistant pharmaceutically active ingredient present both in compositions (A) and (B). Preferably, the amount of the acid resistant pharmaceutically active ingredient present in composition (B) is equal to or smaller than the amount of the same acid resistant pharmaceutically active ingredient present in composition (A).
Preferably, the ratio of distribution of the acid resistant pharmaceutically active ingredient between composition (A) and (B) is about 66% to about 33% or about 50% to about 50%, particularly in the case where the acid resistant active ingredient is amoxicillin and the acid labile pharmaceutically active ingredient present in composition (B) is clavulanic acid.

In case the acid resistant pharmaceutically active ingredient is distributed between composition (A) and (B) in a ratio as herein described, said acid resistant pharmaceutically active ingredient present in composition (B) may be in a different form, e.g. in a different salt or ester form, as compared to the acid resistant pharmaceutically active ingredient present in composition (A). For example, composition (A) may comprise amoxicillin in the form of a sodium salt, and composition (B) may comprise amoxicillin in its trihydrate form, in addition to clavulanic acid, e.g. in the form of the potassium salt.

The kit of parts of the invention is to be administered in such a way that composition (B) is to be administered before composition (A) wherein administration of (B) and (A) is to be separated by a meal. Composition (B) is to be administered before a meal, and composition (A) is to be administered after a meal. "Before a meal" as herein used, particularly in context with the administration of composition (B), is understood to mean some minutes before a meal, for example about 5 to 30 minutes before a meal, or directly at the beginning of a meal, e.g. before a human subject, e.g. a patient is initiating ingestion of a meal, or even when said human subject is about to initiate ingestion of a meal, e.g. after the first bite of a meal. "After a meal" as herein used, particularly in context with the administration of composition (A), is understood to mean about 10 to 100 minutes, such as 20 to 60 minutes, e.g. 30 minutes after the ingestion of a meal.

Alternatively, composition (A) may be administered during the meal, such as about 20 to 40 minutes after a human subject has initiated ingestion of a meal, or at least at a time when the greater part of the meal has already been ingested.

The meal which is used to separate the administration of compositions (A) and (B) may be any amount of food such as a breakfast, a snack, lunch, dinner or any other food intake. Said meal may further be a light or heavy meal, e.g. may be poor or rich in calories, e.g. rich in fat, or may be poor or rich in carbohydrates. The meal may be a standardized high-fat meal, e.g. may be a high-fat meal or breakfast comprising 2 eggs fried in butter, two strips of bacon, two slices of toast with butter, 120 ml (four ounces) of hash brown potatoes and 240 ml (eight ounces) of whole milk. Alternatively, the meal may be a standard light meal, e.g. rich in carbohydrates, e.g. may be a breakfast consisting of 2 slices of bread, 40 g cheese, 150 ml orange juice, 150 ml milk, and 20 g of cereals.

In one embodiment, the kit of parts of the invention comprises composition (A) comprising an acid resistant β-lactam, e.g. an acid resistant penicillin, and composition (B) comprises an acid labile β-lactam compound, preferably being a β-lactamase inhibitor.

In one preferred embodiment, the kit of parts of the invention comprises composition (A) comprising amoxicillin and composition (B) comprising clavulanic acid. Preferably, said kit of parts comprises composition (A) comprising amoxicillin or one of its pharmaceutically acceptable salts and/or solvates and/or hydrates, preferably as the only pharmaceutically active ingredient, and composition (B) comprising clavulanic acid or one of its pharmaceutically acceptable salts and/or solvates and/or hydrates and amoxicillin or one of its pharmaceutically acceptable salts and/or solvates and/or hydrates, preferably as the only pharmaceutically active ingredients. More preferably, said kit of parts of the invention comprises composition (A) comprising amoxicillin in the form of the sodium salt, preferably as the only pharmaceutically active ingredient, and composition (B) comprising clavulanic acid in the form of the potassium salt and amoxicillin in the form of the trihydrate, preferably as the only pharmaceutically active ingredients, wherein compositions (B) is preferably an immediate release formulation. Example 1 shows the components of such a kit of parts of the invention.

In one preferred embodiment of the kit of parts of the invention, composition (A) comprises 50% - 90%, e.g. 60 % - 85%, such as 70 % - 85%, preferably about 72% w/w of amoxicillin, preferably in the form of amoxicillin sodium, as the only pharmaceutically active ingredient, and composition (B) comprises 20 % - 50%, e.g. 30% - 40%, e.g. about 30% - 35%, preferably about 31% w/w of amoxicillin, preferably in the form of amoxicillin trihydrate, and 10% - 30%, e.g. 12% - 25%, such as 15% - 19%, preferably about 15 % w/w of clavulanic acid, preferably as potassium clavulanate, as the only pharmaceutically active ingredients, wherein % w/w are weight/weight percentages relating to the total weight of the pharmaceutical composition (A) or of the pharmaceutical composition (B).

In one embodiment the kit of parts of the invention comprises both composition (A) and composition (B) within one package wherein composition (A) and composition (B) are provided in separate containers. In an alternative embodiment the kit of parts of the invention comprises both composition (A) and composition (B) within one package wherein composition (A) and composition (B) are provided in the same container, for example within one blister, but as separate entities therein, e.g. being each located in separated blister compartments. Said container may a blister, e.g. an aluminium blister, or a bag, e.g. an aluminium bag, or a bottle, e.g. a glass or plastic bottle, e.g. a polyethylene bottle, such as known as securitainer.

The kit of parts of the invention preferably provides composition (A) and composition (B) together with instructions for separate or sequential administration. Sequential administration is to be understood to mean that composition (B) is to be administered before composition (A) in such way that said administration of both compositions is separated by a meal. Thus instructions will indicate that composition (B) is to be administered before a meal and composition (A) is to be administered after a meal.

Alternatively, the kit of parts of the invention may provide in addition to composition (A) and composition (B) a third composition (C), or a forth composition (D), or even further compositions (E), (F) etc. Said additional compositions are characterized in that they comprise either an additional acid resistant or an additional acid labile active ingredient, preferably as the only active ingredient, in admixture with pharmaceutically acceptable excipients as herein described. Said kits of parts are characterized in that all compositions comprising an acid labile active ingredient are to be administered before a meal, and that all compositions comprising an acid resistant active ingredient as the only active ingredient are to be administered after a meal as herein described. It is however, preferred, that the kit of parts of the invention comprises preferably within one package a total of 4, more preferably of 3, most preferably of 2 different compositions wherein one of them comprises one acid labile and the other one comprises one acid resistant active ingredient.

The kit of parts of the invention may be used as a medicament. The disease to be prevented and/or treated will define which type of acid resistant active ingredient will be comprised in composition (A) and optionally also in composition (B), and which type of acid labile active ingredient which will be comprised in composition (B). Said disease will thus define the combination of the acid resistant and the acid labile active ingredient and consequently also the combination of the pharmaceutical compositions (A) and (B) used in the kit of parts of the invention. The person skilled in the art of pharmacological therapy will know which combinations of acid labile and acid resistant active ingredients are therapeutically effective and useful. The term "therapeutically effective" as herein used is understood to mean to provide a desirable therapeutical, prophylactical, physiological and/or pharmacological and/or antimicrobial, e.g. antibacterial, effect.

The kit of parts of the invention comprises the acid resistant and acid labile active ingredients in therapeutically effective amounts.

Examples for therapeutically effective combinations of an acid labile active ingredient and an acid resistant active ingredient are e.g. the combination of amoxicillin and clavulanic acid, or a combination comprising pantoprazole, amoxicillin and clarithromycin.

A kit of part of the invention comprising amoxicillin and clavulanic acid such as herein described, e.g. in Example 1, may be used for the treatment of infectious diseases such as infections of the upper and lower respiratory tract, e.g. pneumonia, infections of the middle ear, nose and throat, skin and soft tissue infections, urinary tract infections and others.

The kit of parts of the invention may be used for the manufacture of a medicament for the prevention and/or treatment of a disease being susceptible to the combination of the acid resistant active ingredient and the acid labile active ingredient therein comprised. "Being susceptible to the combination of the acid resistant active ingredient and the acid labile active ingredient" is understood to mean that said disease will be prevented and/or cured and/or the symptoms of said disease will be relieved, controlled or eliminated by the combined action of the above mentioned active ingredients.

The present invention also relates to the use of a meal for separating the oral administration of a first composition (A) comprising an acid resistant pharmaceutically active ingredient in admixture with a pharmaceutically acceptable excipient, from a second composition (B) comprising an acid labile pharmaceutically active ingredient in admixture with a pharmaceutically acceptable excipient wherein compositions (A) and (B) are not identical and composition (B) is to be administered before composition (A).

In another aspect of the invention, there is provided a method for preventing and/or treating a disease being susceptible to the combination of an acid resistant active ingredient and an acid labile active ingredient wherein administration of a first pharmaceutical composition (A) comprising an acid resistant pharmaceutically active ingredient in admixture with a pharmaceutically acceptable excipient is separated by a meal from the administration of a second pharmaceutical composition (B) comprising an acid labile pharmaceutically active ingredient in admixture with a pharmaceutically acceptable excipient wherein compositions (A) and (B) are not identical. This separation of the administration of the 2 compositions is done in such way that composition (B) is administered before the meal and composition (A) is administered after the meal.

The method of prevention and/or treatment of a disease being susceptible to the combination of an acid resistant active ingredient and an acid labile active ingredient comprises administering to a mammal, preferably to a human patient, a kit of parts comprising compositions (A) and (B) of the invention according to the way as herein described. Thus, said method comprises administering to e.g. a human patient, the kit of parts comprising composition (A) comprising the acid resistant pharmaceutically active ingredient in admixture with a pharmaceutically acceptable excipient; and composition (B) comprising the acid labile pharmaceutically active ingredient in admixture with a pharmaceutically acceptable excipient, which compositions (A) and (B) are not identical, in such way that composition (B) is administered before composition (A), and administration of (B) and (A) is separated by a meal as herein described.

The kit of parts of the invention advantageously allows to administer an acid resistant active ingredient in combination with an acid labile active ingredient in such way to ensure optimum bioavailability of both active ingredients despite food intake. This is in contrast to known combinations of the same active ingredients, such as an extended release tablet comprising amoxicillin and clavulanic acid, marketed under the trade name AUGMENTIN^{™} XR by Glaxosmithkline, wherein the bioavailability of the acid labile active ingredient, in this case clavulanic acid, is reduced by food intake as seen in Figure 1 and in Comparative Example A.

As a further advantage, the kit of parts of the invention, especially when administered according to the invention as defined in the claims and as herein described, allows to provide high bioavailability reflected by a high AUC (area under the plasma concentration time curve) and by long lasting blood levels of the acid resistant active ingredient due to a slow and/or prolonged release of said ingredient from composition (A) accompanied by shorter lasting and more rapidly obtained blood levels of the acid labile active ingredient present in composition (B) which may be desired for obtaining therapeutic efficacy of the combination of the two active ingredients. This advantage is even more clearly expressed when composition (A) comprises a first part of the acid resistant active ingredient in an extended release formulation and when composition (B) comprises the acid labile active ingredient and the second part of the acid resistant active ingredient in an immediate release formulation as herein described. Said advantage is especially interesting when composition (A) comprises amoxicillin or one of its pharmaceutically acceptable salts and/or solvates and/or hydrates, preferably amoxicillin as sodium salt, preferably as the only active ingredient, and when composition (B) comprises clavulanic acid or one of its pharmaceutically acceptable salts and/or solvates and/or hydrates, preferably clavulanic acid as potassium salt, together with amoxicillin or one of its pharmaceutically acceptable salts and/or solvates and/or hydrates, preferably amoxicillin as trihydrate, preferably as the only active ingredients, and is an immediate release formulation such as seen in Example 1.

Without wishing to be bound by any mechanism or theory the inventors believe that those advantages are obtained due to the fact that the administration of the acid labile active ingredient, e.g. of clavulanic acid, prior to food intake assures that said active ingredient resides only for a relatively short term in the stomach and that it is therefore only shortly exposed to the damaging influences of the acidic environment therein. Furthermore, administration prior to food intake seems to make the acid labile active ingredient pass on rapidly due to fast emptying of the stomach which leads to a fast absorption. As a combined effect, the absorption of the acid labile active ingredient being administered before a meal is more complete and faster when compared to the absorption after a meal. On the other hand, the administration of the acid resistant active ingredient after the meal will provide a higher bioavailability and lead to a slightly slower and delayed absorption leading to prolonged therapeutically effective blood levels when compared to administration before the meal. This absorption delaying effect may be further enhanced by administering the acid resistant active ingredient comprised in an extended release formulation. These effects are believed to be due to an optimum intragastric localization of either composition (B) or composition (A) when administered as herein described and claimed which again seems to positively influence the disintegration and/or dissolution behaviour of the compositions and consequently the absorption of both active ingredients.

In a preferred embodiment wherein the acid resistant active ingredient of composition (A) is also partly present in composition (B) as herein described and is thus administered together with the acid labile active ingredient before food intake, one may expect therapeutically effective blood levels of both active ingredients following an initial rapid absorption of both active ingredients as well as prolonged blood levels of the acid resistant active ingredient due to the slower absorption thereof after food intake. This effect is particularly desired in case that the kit of parts of the invention comprises amoxicillin and clavulanic acid, for example as described in Example 1.

As described in detail below, the inventors have investigated the gastrointestinal transport and the in vivo disintegration behaviour of a conventional combination of an acid labile and an acid resistant active ingredient, namely of clavulanic acid and amoxicillin, in the form of a magnetically marked extended release tablet - herein also called test tablet - when administered under fasted or fed conditions, or at the beginning of a meal by applying the Magnetic Marker Monitoring technique. They have also investigated the absorption rate of amoxicillin and clavulanic acid under the same conditions. They have found that the administration of the test tablet before a meal, after a meal or at the beginning of a meal has not been able to provide the desired bioavailability of both of the active ingredients expected to ensure optimum therapeutic efficacy.

The present inventors have now surprisingly found that food intake and the therewith related activities of the stomach such as gastric emptying behaviour may be used to optimize absorption conditions for both the acid labile and the acid resistant active ingredient thus improving the bioavailability of both. They have thus developed the kit of parts of the invention which is to be administered as described herein and in the claims.

The present invention is illustrated by way of the Examples below, but in no way limited to them:

### Example 1:

### Composition B:

in form of a tablet comprising 250 mg amoxicillin as trihydrate and 125 mg clavulanic acid as clavulanate potassium salt and to be administered before a meal or at the beginning of a meal:

| | Amount in mg per tablet |
|---|---|
| Tablet Core | |
| Amoxicillin trihydrate | 291 |
| Clavulanate potassium | 149 |
| Sodium starch glycolate | 5 |
| Microcrystalline cellulose | 291 |
| Magnesium stearate | 9 |
| Silicon dioxide colloidal | 5 |
| Total Core | 750 |
| | |

| Film-Coating | |
|---|---|
| Titan dioxide | 20 |
| Talc | 16 |
| Ethylcellulose | 12 |
| Hydroxypropyl methylcellulose | 10 |
| Triethylcitrate | 2 |
| Total Tablet | 810 |

### Composition A:

in form of a tablet comprising 625 mg amoxicillin as sodium salt and to be administered after a meal:

| | Amount in mg per tablet |
|---|---|
| Tablet Core | |
| Amoxicllin sodium salt | 728 |
| Sodium starch glycolate | 25 |
| Silicon dioxide colloidal | 11 |
| Magnesium stearate | 18 |
| Microcrystalline cellulose | 39 |
| Total Core | 821 |
| | |

| Film-Coating | |
|---|---|
| Titan dioxide | 16 |
| Talc | 14 |
| Ethylcellulose | 9 |
| Hydroxypropyl methylcellulose | 9 |
| Triethylcitrate | 2 |
| Total Tablet | 871 |

### Preparation of Tablet B:

Amoxicillin is granulated with water in an extruder and dried in a fluid-bed dryer. The dry granules are sieved with clavulanate potassium, sodium starch glycolate, microcrystalline cellulose, colloidal silicon dioxide and magnesium stearate, and mixed in a gravity mixer. This mixture is pressed to tablets on an common rotary tabletting pres s to 7x18 mm oblong tablets. Titan dioxide, talc, hydroxypropyl methylcellulose, triethylcitrate and a water based dispersion of ethylcellulose are dissolved and suspended in water. This suspension is sprayed onto the tablets in an conventional film-coater such as an Accela Cota. The tablets are subsequently dried and packed in High Density Polyethylene (HDPE) bottles with dessicant bags containing commercially available molecular sieves such as molecular sieve 4A as commercially available from Multisorb Technologies Inc.

### Preparation of Tablet A:

Amoxicillin is granulated with water in an extruder and dried in a fluid-bed dryer. The dry granules are sieved with sodium starch glycolate, microcrystalline cellulose, colloidal silicon dioxide and magnesium stearate, and mixed in a gravity mixer. This mixture is pressed to tablets on an common rotary tabletting press to 9x19 mm oblong tablets. Titan dioxide, talc, hydroxypropyl methylcellulose, triethylcitrat and a water based dispersion of ethylcellulose are dissolved and suspended in water. This suspension is sprayed onto the tablets in an conventional film-coater such as an Accela Cota. The tablets are subsequently dried and packed in HDPE bottles.

Subsequently the HDPE bottles containing Tablets A and those containing Tablets B are put together into one package and are supplemented with a package insert comprising instructions for administration of Tablet B before a meal or at the beginning of a meal, and for administration of Tablet A after a meal.

### Comparative Example A:

### Influence of food intake on absorption of clavulanic acid and amoxicillin administered in combination

An exploratory study involving 9 healthy volunteers receive the test product under fasted and fed conditions. The test product is AUGMENTIN^{™} XR as commercially available by Glaxosmithkline, and is an extended release tablet containing 1000 mg amoxicillin and 62.5 mg clavulanic acid wherein said tablet consists of a fast release layer comprising 562.5 mg as amoxicillin trihydrate and 62.5 mg clavulanic acid as clavulanate potassium salt, and of an extended release layer containing 437.5 mg amoxicillin as amoxicillin sodium salt. The test tablets are magnetically marked as described in Comparative Example B. The volunteers receive one test tablet together with 240 ml of non-carbonated water under the following conditions:
a) under fasting conditions: on one study day after a fasting period of at least 8 hours,
b) at the beginning of a meal: on another study day after the first bite of a high carbonate standardized breakfast A, and
c) under fed conditions: on still another study day 30 minutes after a high fat standardized breakfast B.
   The standardized breakfast A consists of 2 slices of bread, 40 g cheese, 150 ml orange juice, 150 ml milk, and 20 g of cereals. The standardized breakfast B consists of 2 eggs fried in butter, 2 strips of bacon, 2 slices of toast with butter, 120 ml of hash brown potatoes and 240 ml of whole milk (3.5 % fat).
   Blood samples are taken before and 0.5, 1, 1.5, 2, 2.5, 3, 3.5, 4, 4.5, 5, 5.5, 6, 6.5, 7, 7.5, 8, 8.5, 9, 9.5, 10 and 12 hours after ingestion of the test tablet. Blood samples are centrifuged and stored at -80°C. Concentrations of both amoxicillin and clavulanic acid are determined according to known methods using a LC-MS/MS procedure (HPLC coupled Online with MS). Pharmacokinetic parameters are expressed as Cₘₐₓ (maximal plasma concentration), AUC_{0→∞} (area under the plasma concentration-time curve extrapolated to infinity) and tₘₐₓ (time of peak plasma concentration). Cₘₐₓ and tₘₐₓ as figuring in Tables 1 and 2 are determined from the 9 single values directly taken from the plasma-concentration-time curve. AUC_{0→∞} is calculated as follows: AUC_{0→∞} = AUC_{0→tlast} + AUC_{tlast→∞} wherein AUC_{0→tlast} is the area under the plasma concentration-time curve from time of administration until time of last quantifiable concentration measured, i.e. Cₜ, and is calculated by using the linear trapezoid rule. AUC_{tlast→∞} is the extrapolated area to infinity that equals to Cₜ/Kₑₗ wherein Kₑₗ is the elimination rate constant. The results are reported using descriptive statistics.

Figure 1 shows the plasma concentrations of clavulanic acid under fasting and fed conditions, and after administration at the beginning of a meal (Y-axis: plasma-concentrations of clavulanic acid in µg/ml; X-axis: time in minutes after administration of the test tablet). Figure 1 shows that plasma concentrations of clavulanic acid are significantly reduced after food intake, i.e. when the tablet is taken after a meal, such as the standardised breakfast B, due to reduced absorption of clavulanic acid. Administration of the test tablet at the beginning of a meal leads to plasma levels of clavulanic acid being similar to those observed under fasting conditions.

Figure 2 shows plasma concentrations of amoxicillin under fasting and fed conditions, and after administration of the tablet at the beginning of a meal (Y-axis: plasma-concentrations of amoxicillin in µg/ml; X-axis: time in minutes after administration of the test tablet). Figure 2 shows that food intake significantly influences plasma concentrations of amoxicillin by causing a delayed absorption under fed conditions as compared to fasting conditions. Administration of the test tablet after food intake seems to lead to slightly lower maximum plasma concentrations Cₘₐₓ but to prolonged plasma levels which seem to be high enough to be therapeutically effective. Administration of the test tablet at the beginning of a meal leads to plasma levels of amoxicillin that are similar to those observed under fasting conditions, but the plasma concentration-time curve shows a slight delay of absorption. Said slight delay, reflected by a slight shoulder on the trailing end of the peak does not, however, result in the prolonged higher plasma levels as observed under fed conditions.

Tables 1 and 2 show the pharmacokinetic parameters of clavulanic acid and amoxicillin, respectively, determined in human plasma from the blood samples taken after administration of one test tablet under fasting or fed conditions or at the beginning of a meal (values are mean values of 9 volunteers).

**Table 1: Pharmacokinetik parameters of clavulanic acid**

| | AUC_{0→∞} [µg•min/mL] | Cₘₐₓ [µg/mL] | tₘₐₓ [min] |
|---|---|---|---|
| Administration under fasting conditions | | | |
| Mean value ± s | 198 ± 48 | 1.40 ± 0.4 | 90 ± 37 |
| Median | 181 | 1.35 | 90 |
| Administration under fed conditions | | | |
| Mean value ± s | 132 ± 71 | 0.97 ± 0.6 | 124 ± 41 |
| Median | 125 | 0.83 | 120 |
| Administration at the beginning of a meal | | | |
| Mean value ± s | 197 ± 43 | 1.39 ± 0.4 | 83 ± 25 |
| Median | 173 | 1.24 | 90 |

Table 1 shows that the pharmacokinetic parameters Cₘₐₓ and AUC of clavulanic acid are significantly reduced in a fed state as compared to fasting conditions. As these parameters are indicative of absorption and of bioavailability of a given active ingredient, these findings show absorption and hence bioavailability of clavulanic acid are decreased when the test tablet is administered in a fed state. When the test tablet is administered at the beginning of a meal, Cₘₐₓ and AUC are similar to those observed under fasting conditions.

**Table 2: Pharmacokinetic parameters of amoxicillin**

| | AUC_{0→∞} [µg•min/mL] | Cₘₐₓ [µg/mL] | tₘₐₓ [min] |
|---|---|---|---|
| Administration under fasting conditions | | | |
| Mean value ± s | 1854 ± 280 | 10.3 ± 1.2 | 90 ± 26 |
| Median | 1740 | 10.3 | 90 |
| Administration under fed conditions | | | |
| Mean value ± s | 2604 ± 446 | 9.88 ± 2.3 | 137 ± 83 |
| Median | 2526 | 9.83 | 90 |
| Administration at the beginning of a meal | | | |
| Mean value ± s | 2454 ± 355 | 10.4 ± 2.0 | 83 ± 33 |
| Median | 2346 | 9.87 | 60 |

The values for AUC of amoxicillin are significantly reduced when the test tablet is taken under fasting conditions as compared to fed conditions indicating that absorption and hence bioavailability of amoxicillin are decreased as compared to administration in a fed state. When the test tablet is administered at the beginning of a meal, AUC of amoxicillin lies in between that observed for the fasting and for the fed state. tₘₐₓ is significantly delayed under fed conditions as compared to fasting conditions or to administration at the beginning of a meal.

### Comparative Example B:

### Influence of food intake on gastrointestinal localization and transit as well as on disintegration behaviour of magnetically marked extended release tablets comprising amoxicillin and clavulanic acid

Within the exploratory study described in Comparative Example A, the same 9 healthy volunteers receive the same test product under the same 3 conditions described as a), b) and c). Both layers of the test tablet have been magnetically labeled along their short axis using magnetite, i.e. black iron oxide Fe₃O₄, a commercially available colouring agent which is commonly used in food and pharmaceutical industry and which is approved by European authorities as E 172. The magnetic labeling is performed by making a drill hole on both sides of each tablet, i.e. each tablet is magnetically labeled in the fast release layer and in the extended release layer. Each drill hole contains 6 mg ± 1 mg of a mixture of 90% magnetite and 10% microcrystalline cellulose; the aperture of the drill hole in the extended release layer is additionally closed with magnesium stearate. Both microcrystalline cellulose and magnesium stearate are excipients already comprised in the test tablet. Prior to ingestion the tablets are magnetized along their short axis for 5 minutes in a homogenous magnetic field of at least 1 Tesla by using an electromagnet (BE 10 V, Bruker, Germany). The magnetic moments of the individual iron oxide particles are thereby aligned. As a result the aligned particles generate, as an ensemble, a net magnetic moment, i.e. the tablet is transformed into a stable magnetic dipole. After contact with liquids e.g. with gastro-intestinal fluids, the magnetic particles are released from the tablets due to erosion and/or swelling processes and thus lose their alignment which results in a decrease of the magnetic moment over time. After having swallowed the magnetically labeled tablet the volunteer is placed in supine position under a biomagnetic measurement device that is operated in a magnetically shielded standard environment for recording of the magnetic field outside the body. This device contains 83 superconducting interference devices (SQIDs), i.e. highly sensitive magnetic field sensors, combined electronically to 63 gradiometer channels and a data acquisition system recording the magnetic field distribution over an area of 230 mm diameter at a sampling rate of 250 Hz as described analogously in Müller W., G.P., Riedel W., Burghoff M., "Mehrkanal-Aufnahmesystem für Biosignale mit bis zu 512 analogen Ergänzungen." Biomedizinische Technik (2003) 48 (Ergänzungsband 1). The 83 SQUID gradiometer system used in the study is available at the Physikalisch Technische Bundesanstalt, Benjamin-Franklin-Hospital of the Charite Berlin, Germany. Measurement of localization is performed analogously to the method of Magnetic Marker Monitoring as described in Weitschies W. , Wedemeyer R.-S., et al, "Impact of the intragastric location of extended release tablets on food interactions", Journal of Controlled Release, 108 (2005) 375-385. Instead of applying only the magnetometer mode, the system is additionally operated in a gradiometer mode wherein multiple sensors are coupled to suppress distant disturbing signals. At intervals of 30 minutes, the magnetic field is recorded for 5 minutes starting immediately after ingestion of the test tablet. During measurement the sensor is positioned at 1 to 5 cm from the abdomen of the volunteer. Between each recording the volunteer is allowed to walk around, each hour the volunteer drinks 150 ml non-carbonated water. For lunch the volunteers receive a standardized meal consisting of chicken fricassee and 240 ml non-carbonated mineral water. The measurements of the magnetic field continue until the net magnetization is less than about 5 to 10 % of the initial magnetic signal. Localization of the test tablet is performed by using a coordinate system originating at the jugulum of the volunteer with the x-axis pointing to the volunteer's left, the y-axis pointing to the cranial direction and the z-axis pointing in the ventral direction. Data of localization are filtered digitally and reduced to a frequency of 50 Hz as described analogously in Weitschies W., Karaus M., et al., "Magnetic Marker Monitoring of disintegrating capsules", European Journal of Pharmaceutical Sciences 13 (2001) 411-416, and Weitschies W., Cardini D. et al., "Magnetic marker monitoring of esophageal, gastric and duodenal transit of non-disintegrating capsules", Pharmazie, 54 (1999) 6, pages 426-430. Position and disintegration state are determined by fitting the field of a magnetic dipole to the measurement data in inverse field calculation using the Levenberg-Marquart-Algorithm as described in Press W.T., SA, Vetterling, WT; Flannery, BP, "Numerical recipes in C". Cambridge University Press (1995) p. 681-688. Further data evaluation is performed using the program "Axum 5.0 C for Windows" (MathSoft, Inc.).Tablets are localized visually using x-y- and x-z-localization diagrams which includes comparing the motion and coordinates of the tablet with normal anatomy e.g. tracing the typical C-like movement through the duodenum in the x-y plane, as described in Weitschies W. , Wedemeyer R.-S., et al (2005, see above). In this way the magnetic moment of the tablet together with the associated localization of the tablet has been tracked.

The time endpoints of gastric emptying are determined by visual control of gastrointestinal localization of the test tablet. If gastric emptying takes place in between 2 measurements, the arithmetic mean of the 2 measuring times is taken as point of time of gastric emptying.

Table 3 below shows gastric emptying times in minutes of the magnetically labeled test tablet when administered under fasting or fed conditions, of when administered at the beginning of a meal (p1 to p9 are the 9 individual volunteers):

| Gastric emptying (minutes) | Administration under fasting conditions | Administration at the beginning of a meal | Administration under fed conditions |
|---|---|---|---|
| p1 | 14 | * | * |
| p2 | 185 | 370 | * |
| p3 | 45 | 190 | * |
| p4 | 25 | 210 | * |
| p5 | 160 | * | 311 |
| p6 | 17 | 174 | 290 |
| p7 | 30 | 195 | 300 |
| p8 | 35 | 190 | * |
| p9 | 50 | * | * |
| mean ± S | 62 ± 60 | 221 ± 76** | 300 ± 9*** |
| median | 35 | 193 | 300 |

| | | | |
|---|---|---|---|
| * disintegration of the tablet is completed in the stomach *** n = 3 (i.e. without p1, p2, p3, p4, p8, p9) ** n = 7 (without p1, p5, p9) | | | |

Under fasting conditions the tablets are emptied from the stomach of all volunteers after a mean gastric residence time of 62 minutes. After administration of the tablet at the beginning of a meal tablets are already fully disintegrated within the stomach of 3 volunteers. Under fed conditions the test tablets are fully disintegrated in the stomach in 6 out of 9 individuals.

Disintegration of the test tablet is indicated by the gradual disappearance of the signal for the magnetic moment until it is no longer detectable which then indicates complete disintegration, and also the end of measurements for localization. Table 3 clearly shows that gastric emptying time is prolonged by food intake.

Table 4 below shows the decay times of the magnetic markers in the fast release layer, i.e. layer 1 and in the extended release layer, i.e. layer 2, of the test tablet after administration under fasting and fed conditions (p1 to p9 are the 9 individual volunteers):

| | Administration under fasting conditions | | Administration under fed conditions | |
|---|---|---|---|---|
| Decay time (minutes) | Layer 1 | Layer 2 | Layer 1 | Layer 2 |
| p1 | 16 | 162.1 | 39.9 | 175.3 |
| p2 | 23.5 | 159.9 | 11.4 | 223.2 |
| p3 | 27.1 | 174.3 | 19.4 | 116.4 |
| p4 | 16.9 | 175.5 | 38.7 | 277.5 |
| p5 | 14.9 | 146.2 | 63.4 | 384.7 |
| p6 | 19.6 | 114.2 | 21.1 | 232.7 |
| p7 | 45.8 | 220.1 | 82 | 281.4 |
| p8 | 49.2 | 171.8 | 15.5 | 203.8 |
| p9 | 35 | 204.7 | 15.8 | 231.4 |
| mean ± S | 27 ± 12 | 169 ± 29 | 34 ± 23 | 236 ± 70 |
| median | 23.5 | 171.8 | 21.1 | 231.4 |

Values of Table 4 are decay times in minutes of the magnetic markers of the 2 layers of the magnetically labeled test tablet. More particularly, decay times for the fast or immediate release layer (layer 1) indicate the endpoint of the decay of the magnetic marker in layer 1; and decay times for the extended release layer (layer 2) indicate the beginning of the decay of the magnetic marker of layer 2. The point of time of decay of the 2 layers is determined by linear regression from the magnitude of the magnetic moments of the single measurements. The fast release layer disintegrates quicker under fasting conditions than under fed conditions. Food intake additionally seems to cause a slightly higher variability of the disintegration behaviour shown as a broader range of decay times among the individual volunteers. When the test tablet is administered at the beginning of a meal, the fast release layer disintegrates in about the same time as under fasting conditions (data not shown). Food intake seems to delay disintegration of the extended release layer of the test tablet comprising amoxicillin when compared to the fasting state. This indicates that tablet erosion in the stomach is reduced under fed conditions when compared to fasting conditions. Administration at the beginning of a meal also seems to lead to a delayed disintegration of the extended release layer (data not shown).

### Gastrointestinal localization and transit of the test tablets:

After administration under fasting conditions the test tablet shows a fast passage through the stomach which is reflected by gastric emptying times lying between 14 minutes and 50 minutes for most volunteers; only 2 test persons have shown longer gastric emptying times of about 160 or 185 minutes (see Table 3). Gastric residence time of the tablet is thus relatively short. Additionally, the tablets show considerable movements within the stomach, duodenum and small intestines before their magnetic markers totally decay within about 120 minutes to about 220 minutes which marks the end of the measurement of localization.

After administration under fed conditions, i.e. after standardized breakfast A, the test tablet in most volunteers initially stays in the proximal part of the stomach, i.e. in the fundus, for a relatively long period of time before moving towards the distal part of the stomach, i.e. the antrum. For most volunteers, the tablet has fully disintegrated before leaving the stomach (see Table 3). The markers within the test tablets fully decay after about 150 minutes to about 360 minutes which ends all measurements of localization.

After administration at the beginning of a meal, i.e. with the first bite of breakfast B, the test tablet shows an initial residence in the distal part, i.e. antrum, or in the corpus of the stomach during the first magnetic measurement, and in most volunteers it stays there for at least about 60 minutes. In some volunteers the tablet stays in the antrum until it leaves the stomach, in other volunteers it moves "back" to the corpus or fundus of the stomach before gastric emptying takes place. The markers of test tablets fully decay between 180 minutes and about 360 minutes which marks the end of the localization measurement. Gastric emptying times are prolonged so that the tablet has fully disintegrated in the stomach in 3 volunteers (see Table 3).

### Summary and interpretation of the results of Comparative Example A and B:

After administration under fasting conditions, the test tablets are emptied from the stomach after a mean residence time of 62 minutes which is significantly faster than under fed conditions or when administered at the beginning of a meal. The fast release layer disintegrates after about 27 minutes which correlates with a rapid increase in amoxicillin plasma levels. Maximum amoxicillin plasma levels are observed in about 90 minutes on an average. Under fasting conditions, the amoxicillin plasma concentration time-curve shows a single peak caused by the disintegration of the first layer. The bioavailability of amoxicillin, i.e. mean AUC, is significantly lower as compared to fed conditions or after administration of the tablet at the beginning of a meal which the inventors assume to be due to the existence of an absorption window in the upper part of the small intestine combined with the effect of a rapid gastric emptying. Maximum plasma levels of clavulanic acid are obtained within about 90 minutes on an average. Mean AUC of clavulanic acid is significantly higher under fasting conditions as compared to fed conditions. This seems to be due to the fact that either the whole tablet as such or the dissolved portion of the fast release layer are rapidly emptied from the stomach. With increasing gastric residence time, such as under fed conditions, AUC of clavulanic acid shows a tendency to decrease.

After administration of the test tablet at the beginning of a meal, i.e. after the first bite of e.g. a standardized breakfast, the test tablet is initially localized in the distal part of the stomach in all individuals tested. Presumably, the test tablet is first located in the fundus and then pushed towards the antrum by the food being subsequently ingested. Maximum amoxicillin plasma levels are obtained in about 83 minutes on an average which is comparable to results observed under fasting conditions. Additionally, amoxicillin plasma concentration-time curves show a slight shoulder in the trailing edge of the peak caused by the fast release layer. This shoulder may reflect the absorption of amoxicillin from the extended release layer due to a retentive effect of the stomach. Due to high antral motility after high carbohydrate meals, released amoxicillin is assumed to be well distributed within the stomach and to be released into the duodenum together with portions of the food. Mean AUC of amoxicillin is higher than after administration under fasting conditions, but still lower than observed under fed conditions. Mean AUC of clavulanic acid is comparable to that observed under fasting conditions, but is significantly higher than under fed conditions, because of the early localization of the tablet in the distal stomach which results in a rapid gastric emptying of clavulanic acid being released after the decay of the fast release layer. Thus clavulanic acid is only shortly exposed to the damaging acidic environment of gastric juices.

After administration under fed conditions, the tablet is localized initially in the proximal parts of the stomach and in most volunteers disintegrates fully within the stomach. The test tablet seems to lie on top of the intragastric food and is therefore emptied from the stomach later than in the fasted state - or not at all - due to known gastric motility properties. Prolonged residence times of the tablet in the fundus - as for example observed for volunteers p 1, 4, 5 and 7 (data not shown) - might further lead to incomplete wetting and less mechanical stress. This may explain the distinctly delayed disintegration of the fast release layer observed in those volunteers (see Table 4). Maximum amoxicillin levels are reached at 137 minutes on an average. The amoxicillin plasma concentration-time curve indicates prolonged absorption by showing a distinct shoulder or even a second peak which may partly be due to a lower gastric motility after high fat meals. Mean AUC of amoxicillin is highest under fed conditions when compared to the 2 other regimes. Bioavailability of clavulanic acid, however, is significantly reduced presumably due to longer intragastric residence time of this acid labile active ingredient leading to increased degradation.

### Conclusions:

If a conventional combination of an acid labile active ingredient such as clavulanic acid and of an acid resistant active ingredient such as amoxicillin is administered according to a conventional scheme, namely both active ingredients simultaneously, concomitant food intake seems to strongly influence the plasma concentrations and thus bioavailability of the 2 active ingredients. This has been observed in the herein described exploratory study for a conventional combination in the form of a bilayer tablet comprising clavulanic acid for immediate release and amoxicillin for both immediate and extended release such as the herein described test tablet. If such a conventional combination is administered under fasting conditions, clavulanic acid shows high AUC and thus good bioavailability whereas amoxicillin exhibits decreased bioavailability. If such conventional combination is administered at the beginning of a meal, e.g. after the first bite of a breakfast, clavulanic acid shows similar bioavailability and amoxicillin shows better bioavailability than under fasting conditions. When administered after a meal, such conventional combination leads to a significantly reduced bioavailability of clavulanic acid and to highest bioavailability of amoxicillin. These results have been confirmed by a more extensive study involving 70 volunteers who have swallowed the herein described conventional test tablet - albeit without any magnetic markers - under conditions similar to those herein described. In case the combination is administered at the beginning of a meal, however, a slightly decreased bioavailability for clavulanic acid is observed (data not shown).

The inventors have thus observed that the time point of administration of such a conventional combination of an acid labile active ingredient, such as clavulanic acid, and an acid resistant active ingredient, such as amoxicillin, e.g. in the form of a test tablet as herein described, in relation to the intake of food may have a significant impact on the intragastric tablet localization and on the disintegration behaviour and dissolution characteristics of said tablet. Consequently said time point of administration in relation to food intake may act as a major source of food effects on pharmacokinetics and pharmacodynamics of those 2 active ingredients. These food effects may e.g. undesirably affect absorption and thus bioavailability of the 2 active ingredients. Food intake may also lead to a slightly higher variability of the disintegration time of a solid formulation such as a tablet which is expected to lead to a higher variability of absorption and thus of bioavailability of the therein comprised active ingredients. On the other hand, fasting conditions may also undesirably influence, e.g. reduce the bioavailability of the acid resistant active ingredient, particularly if said active ingredient exists within in an extended release form. Thus the conventional way of administration, i.e. simultaneous administration, before a meal, at the beginning of a meal or after a meal of conventional combinations of an acid resistant and an acid labile active ingredient such as the test tablet described above is not expected to result in a satisfactory bioavailability of both of the active ingredients as is seen above.

On the contrary, the kit of parts of the invention administered as herein described uses food intake in a way to avoid any such undesired effect on the absorption particularly of the acid labile active ingredient, whilst providing on the other hand high bioavailability and prolonged therapeutically effective serum levels of the acid resistant active ingredient, thereby improving the bioavailability of both partners of the combination of an acid resistant and an acid labile active ingredient which is expected to ensure appropriate therapeutic efficacy.

## Claims

1. A kit of parts comprising:
(A) a first pharmaceutical composition comprising an acid resistant pharmaceutically active ingredient in admixture with a pharmaceutically acceptable excipient; and
(B) a second pharmaceutical composition comprising an acid labile pharmaceutically active ingredient in admixture with a pharmaceutically acceptable excipient; which compositions (A) and (B) are not identical,
wherein composition (B) is to be administered before a meal and composition (A) is to be administered after a meal.

2. The kit of parts of claim 1 wherein composition (B) additionally comprises the acid resistant pharmaceutically active ingredient of composition (A).

3. The kit of parts of claim 1 or 2 wherein composition (B) additionally comprises the acid resistant pharmaceutically active ingredient of composition (A) in an amount being equal or smaller than its amount in composition (A).

4. The kit of parts of any one of claims 1 to 3 wherein the acid resistant pharmaceutically active ingredient is an acid resistant β-lactam antibiotic.

5. The kit of parts of claim 4 wherein the acid resistant pharmaceutically active ingredient is amoxicillin.

6. The kit of parts of any one of the preceding claims wherein the acid labile pharmaceutically active ingredient is an acid labile β-lactam compound.

7. The kit of parts of claim 6 wherein the acid labile pharmaceutically active ingredient is clavulanic acid.

8. The kit of parts of any preceding claim wherein composition (A) comprises amoxicillin, or one of its pharmaceutically acceptable salts and/or solvates and/or hydrates, preferably amoxicillin in the form of the sodium salt, and wherein composition (B) comprises clavulanic acid, or one of its pharmaceutically acceptable salts and/or solvates and/or hydrates, preferably clavulanic acid as clavulanate potassium salt, and amoxicillin, or one of its pharmaceutically acceptable salts and/or solvates and/or hydrates, preferably amoxicillin in the form of the trihydrate.

9. The kit of parts of any preceding claim wherein composition (A) comprises about 70% to about 85%, preferably 72 % w/w of amoxicillin, preferably as sodium salt, and composition (B) comprises about 15% to about 19%, preferably about 15 % w/w of clavulanic acid, preferably as potassium clavulanate, and about 30% to about 35%, preferably about 31 % w/w of amoxicillin, preferably as trihydrate, wherein % w/w are weight/weight percentages relating to the total weight of composition (A) or of composition (B).

10. The kit of parts of any preceding claim wherein composition (A) and (B) are in the form of a tablet, capsule, powder for oral suspension, granules for oral suspension, granules, pellets, dispersible tablets or in liquid form such as a suspension.

11. The kit of parts of claim 10 wherein composition (A) and (B) are in the form of a tablet or of a capsule.

12. The kit of parts of claim 10 or 11 wherein composition (A) and (B) are in the same form or in different forms.

13. The kit of parts of any of claims 10 to 12 wherein composition (A) is an extended release formulation, and composition (B) is an immediate release formulation.

14. The kit-of-parts of any preceding claim further providing instructions for sequential administration in such way that composition (B) is to be administered before a meal and composition (A) is to be administered after a meal.

15. The kit-of-parts of any preceding claim wherein composition (A) and composition (B) are comprised within one package.

16. The kit of parts of claim 15 wherein composition (A) and composition (B) are provided in separate containers or in the same container.

17. The kit-of-parts of claim 16 wherein said container is a blister, a bag or a bottle.

18. The kit of parts of any preceding claim for use as a medicament.

19. Use of the kit of parts of any preceding claim for the manufacture of a medicament for the prevention and/or treatment of a disease being susceptible to the combination of the acid resistant active ingredient and the acid labile active ingredient.

20. Use of a meal for separating the oral administration of a first composition (A) comprising an acid resistant pharmaceutically active ingredient in admixture with a pharmaceutically acceptable excipient, from a second composition (B) comprising an acid labile pharmaceutically active ingredient in admixture with a pharmaceutically acceptable excipient wherein compositions (A) and (B) are not identical and composition (B) is to be administered before composition (A).

21. A method for treating a disease being susceptible to the combination of an acid resistant active ingredient and an acid labile active ingredient wherein administration of a first pharmaceutical composition (A) comprising the acid resistant pharmaceutically active ingredient in admixture with a pharmaceutically acceptable excipient is separated by a meal from the administration of a second pharmaceutical composition (B) comprising the acid labile pharmaceutically active ingredient in admixture with a pharmaceutically acceptable excipient wherein compositions (A) and (B) are not identical and composition (B) is to be administered before composition (A).
